# EUROPEAN PATENT SPECIFICATION

(11) **EP 1 960 496 B1**
(45) Date of publication and mention of the grant of the patent: **24.03.2010**
(21) Application number: 06705064.1
(22) Date of filing: 17.01.2006
(51) Int. Cl.: C10G 2/00, C07C 1/12

(54) **A METHOD OF OBTAINING HYDROCARBONS FROM CARBON DIOXIDE**
VERFAHREN ZUM ERHALT VON KOHLENWASSERSTOFFEN AUS KOHLENDIOXID
PROCEDE D'OBTENTION D'HYDROCARBURES A PARTIR DE DIOXYDE DE CARBONE

(30) Priority: 29.07.2005 BG 10924605
(43) Date of publication of application: 27.08.2008
(73) Proprietor: Angelov, Chavdar Angelov, 9000 Varna (BG)
(72) Inventor: Angelov, Chavdar Angelov, 9000 Varna (BG)
(74) Representative: Benatov, Emil Gabriel
(86) International application number: PCT/BG2006/000003
(87) International publication number: WO 2007/012150

(56) References cited:
- EP-A- 0 537 593
- WO-A-01/34538
- WO-A-97/05088
- WO-A-98/57743
- DE-A1- 4 220 865
- DE-A1- 10 156 975
- GB-A- 709 645
- RIEDEL T ET AL: "Comparative study of Fischer-Tropsch synthesis with H2/CO and H2/CO2 syngas using Fe- and Co-based catalysts" APPLIED CATALYSIS A: GENERAL, ELSEVIER SCIENCE, AMSTERDAM, NL, vol. 186, no. 1-2, 4 October 1999 (1999-10-04), pages 201-213, XP004271934 ISSN: 0926-860X
- LEE, M.-D. ET AL.: "Effects of addition of chromium, manganese, or molybdenum to iron catalysts for carbon dioxide hydrogenation" APPLIED CATALYSIS, vol. 72, 1991, pages 267-281, XP009067974

## Description

### FIELD OF APPLICATION

The method of obtaining hydrocarbons from carbon dioxide is applicable to the production of fuels and raw materials for producing chemical products.

### PRIOR STATE OF THE ART

A method of obtaining hydrocarbons by hydrogenating carbon dioxide in the presence of a catalyst containing copper, zinc oxide, zirconium oxide, mixed with a carrier of silicoaluminophosphate SAPO-5, SAPO-34, is known [US6376562]. Preparing the catalyst is performed in the following way:
- Ion exchange of copper nitrate with the carrier of silicoaluminophosphate SAPO-5, SAPO-34;
- Mixing the catalysts obtained by means of ion exchange of copper nitrate with the carrier SAPO-5, SAPO-34, and catalysts for synthesis of methanol like Cu/Zn/ZrO₂, Cr₂O₃/ZnO/Al₂O₃, Pd/SO₂, Cu/Cr₂O₃/ZnO. The process of hydrogenating the carbon dioxide with production of hydrocarbons C₁ - C₆ takes place at temperatures 250 - 500 °C and pressure 25,33 - 35,46 bar (25 - 35 atm).

In the catalysts containing silicoaluminophosphates SAPO-5, SAPO-34, the content of Cu varies from 0.5 to 2 % of the mass. The catalysts, active in the reactions of methanol synthesis, Cu/Zn/ZrO₂, contain from 10 to 62 % of the weight of Cu, from 0 to 30 % of the weight of ZnO, and from 10 to 90 % of the weight of ZrO₂. The ratio of the catalysts for methanol synthesis and the catalysts for methanol conversion varies from 4:1 to 1:4 in weight. The process of preparing the catalysts includes two stages:
- preparing catalysts on the basis of silicoaluminophosphates and catalysts for methanol synthesis;
- mixing the catalysts prepared on the basis of silicoaluminophosphates and the catalysts for methanol synthesis.

Before the reaction of hydrogenating the carbon dioxide, the catalysts are subject to regeneration treatment at temperature 280 °C and atmospheric pressure and at increasing concentration of hydrogen in the argon, starting from 5 volume %, 10, 20, 40, and 100 % of the volume at intervals of 30 min. Then the reactor is filled with helium at a pressure of 28,37 bar (28 atm), after which a mixture of H₂ and CO₂ in the ratio H₂/CO₂ equal to 3 and with volumetric speed 10 to 30 g-cat.h/mol is directed into the reactor, and the temperature is increased to 350 - 400 °C.

The conversion of CO₂ for the catalysts investigated is of the order of 31.8 - 40.2 %, and the hydrocarbons obtained are 9.1 to 15.8 % of the mass. The hydrocarbons obtained feature the following distribution in weight percentage: C₁ - 1.9 to 4 %; C₂ - 11.2 to 34.8 %; C₃ - 25.2 to 52 %; C₄ - 8.8 to 48.5 %; C₅ - 0.7 to 5.0 %; C₆ - 0.1 to 4.0 %.

A disadvantage of the method described is the low quantity of obtained hydrocarbons, their total quantity not exceeding 15.8 % of the mass, and only 2 % of them are liquid. Such a low yield of hydrocarbons makes the method unsuitable to be applied for industrial purposes as it is of low economical efficiency.

The method [US5952540] of producing hydrocarbons by hydrogenating carbon dioxide in the presence of a catalyst containing Fe and K, deposited upon γ-Al₂O₃ with atomic ratio K/Fe in the range 0.1 - 1.5 and content of Fe in the range 5 - 50 % of the catalyst weight, is the closest to the present invention. The catalyst is regenerated with hydrogen and then activated with a mixture of hydrogen and carbon dioxide at temperature 300 - 500 °C, pressure 1,01 - 10,13 bar (1 - 10 atm), and speed of feeding hydrogen 20 - 100 ml/g of catalyst per minute for 24 h. The process of hydrogenating the carbon dioxide takes place at volumetric ratio H₂/CO₂ = 1 - 5, temperature 200 to 500 °C, pressure from 1,01 - 101,32 bar (1 - 100 atm), and volumetric speed 50 - 20,000 h⁻¹. The maximum yield of obtained hydrocarbons attains 47 % of the mass for content of hydrocarbons C₁ - 7.8 %, C₂ and higher - 60.8 %. In this case the content of liquid hydrocarbons does not exceed 10 - 20 % of the mass.

The low yield of obtained hydrocarbons is a disadvantage of this method, which makes it low-efficient for industrial production of gaseous and liquid hydrocarbons.

Hydrogenation of CO, CO2 and their mixtures is described in patent applications published as WO 97/05088 and WO 01/34538 and publication Riedel at al., Applied Catalysis A: General, Vol. 186, no. 1-2 (1999), p. 201-213, according to which the catalysts used are Fe-K/ A1203 and Fe-Cu-K/ A1203. The inventors' interest is focused to the resistance of catalyst to deactivation remaining sufficiently active with the aim- production of C2+ hydrocarbons.

Patent publication WO98/57743 describes a process for incorporation into molecular sieves a catalyst selected from the group consisting of iron, cobalt, nickel, chromium, manganese and rhodium in amount sufficient to catalyze the conversion of synthesis gas to lower olefins. The main object of invention is to find special conditions as to be possible to form complexes of catalyst precursors and molecular sieves with the aim synthesis of lower olefins, which can be manipulated to produce varying amounts of ethylene, propylene, and butenes.

Patent GB709645 investigates the process of preparation of hydrocarbons using as catalysts metals of the 8th group of the periodic system. The decision is focused not to the kind of the metals as such but to its manner of use- suspended in a liquid medium or dry, or dust like catalyst.

Patent application DE 101 56 975 describes a process of production of hydrocarbons from the atmospheric CO2 and water using electric power, especially by nuclear fission or nuclear fusion reactions. Electric power is also on the base of the process according to patent application DE 42 20 865 where the hydrogenation of CO2 with hydrogen atoms for synthesis of methane or methanol is carried out by exposing to a static electric discharge in a reaction zone and where the reaction zone is designed as a device containing electrodes and dielectric substance, containing Cu as a catalyst. The aim of the process is again preparation of low chain hydrocarbons.

EP-A-0537 593 describes a process for recovering carbon dioxide gas present in combustion exhaust gas using absorption a monoethanolamine solution. The decision does not concern the process of hydrogenation of CO2.

Lee, M. -D. et al., Applied Catalysis, vol. 72 (1991), p. 267-281 investigate the iron catalyst behaviour during the hydrogenation of carbon dioxide in the presence of chromium, manganese and molybdenum. The conclusion is that the enhancement of carbide formation in the presence of manganese or molybdenum is responsible for the higher yield of hydrocarbons, but the study is not directed to the entire process of preparation of heavy chain saturated and unsaturated hydrocarbons.

The task of the invention consists in creating a cost-effective method for industrial production of hydrocarbons from carbon dioxide with increased yield of obtained hydrocarbons, including also the liquid C₅ - C₁₀, which are major components of the motor fuels.

### TECHNICAL ESSENCE OF THE INVENTION

This task is solved by creating a method of producing hydrocarbons from hydrocarbon dioxide, including a stage of obtaining carbon dioxide by adsorption with ethanolamine from flue gases with subsequent desorption and mixing with H₂ in volumetric ratio H₂/CO₂ equal to 4, with subsequent compression up to pressure 35,46 - 50,66 bar (35 - 50 atm), heating up to 300 - 350 °C, and passing through a catalyst with volumetric speed 1,500 h⁻¹. The catalyst contains, in percentage of the mass, Fe 40 - 45 %, Mo 1.0 - 2.5 %, K 1.0 - 1.5 %, and a combination of aluminium, zirconium and their oxides and phosphates is used as a carrier. It is possible that gaseous hydrocarbons obtained in the process of hydrogenating the carbon dioxide are subject to catalytic aromatization. It is possible that the gas mixture of H₂ and CO₂ and the catalyst in the process of hydrogenating the carbon dioxide in the first stage are subject to the action of modulated high-frequency fields in the frequency range from 1 MHz to 50 MHz at modulation frequency in the range from 1 KHz to 200 KHz, and that in the second stage they are subject to action by plasma from single-electrode high-frequency discharges in the upper and central parts of the reactor.

An advantage of the method is that it represents a cost-effective method for industrial production of hydrocarbons from carbon dioxide with increased yield of obtained hydrocarbons, including also liquid C₅ - C₁₀, which are major components of the motor fuels.

### EXEMPLARY EMBODIMENT AND OPERATION OF THE INVENTION

The polyfunctional catalyst not only realizes the process of hydrogenating the carbon dioxide in hydrocarbons with high number of carbon atoms, but also exercises oligomerizing activity with respect to the non-saturated hydrocarbons, which permits obtaining from them additional liquid hydrocarbons with greater number of carbon atoms in comparison with the exhausted gaseous hydrocarbons. In addition, the saturated hydrocarbons C₂ - C₄, which are obtained in the process of hydrogenating, are separated from the gas mixture and subject to catalytic aromatization.

To increase the efficiency of catalytic processes, the inletting gas reagents are acted upon by modulated high-frequency fields in the frequency range from 1 MHz to 50 MHz at modulation frequency in the range from 1 KHz to 200 KHz, and then the gaseous reagents are treated with plasma from single-electrode high-frequency discharges.

**Example 1:** The flue gases (CO₂, H₂O, and N₂), discharged from thermal power stations, are introduced into a condenser, where, at temperature 5° C, the water vapor is converted to water. Subsequently, the water is fed back to the water-supply system of the thermal power station. The carbon dioxide and nitrogen enter an adsorber with ethanolamine, in which CO₂ absorption takes place, and N₂ is exhausted to the atmosphere. The ethanolamine containing CO₂ is directed to a desorber, where, at temperature 80 °C, the release of CO₂ is carried out. The carbon dioxide obtained in this way is compressed and directed into a reactor, in which the process of hydrogenating is accomplished at temperature 300 °C and pressure 35,46 bar (35 atm) for volumetric ratio H₂/CO₂ = 4 and volumetric speed 1,500 h^{-\1}. The process takes place in the presence of a catalyst containing (in percentage of the mass) Fe 40 - 45 %, Mo 1.0 - 2.5 %, K 1.0 - 1.5 %. A combination of aluminium, zirconium and their oxides and phosphates is used as a carrier. Before the beginning of the catalytic process, the catalyst is subject to regeneration treatment with a gas mixture containing H₂ and N₂ with step-like increase in the content of H₂, starting from concentration of 5 %. The catalyst regeneration at minimum content of H₂ in the gas mixture is carried out at temperature 450 °C for 72 h with volumetric speed of the gas mixture 250 h⁻¹ at pressure 5,14 bar (5 atm), after which the catalyst is subject to treatment with a mixture of H₂/CO₂ in volumetric ratio equal to 3 at temperature 300 °C, pressure 10,13 bar (10 atm), and volumetric speed 1500 h⁻¹ for 24 h. After terminating the activation process, the pressure in the system is increased up to 35,46 bar (35 atm) and the volumetric ratio of H₂/CO₂ is increased to 4, and the process of hydrogenating the carbon is realized at temperature 300 °C.

The hydrocarbons obtained in these conditions represent 58 % of the mass, and at that the liquid C₅ - C₉ are 22 % of the mass. Besides hydrocarbons, the reaction products contain also 28 % of carbon monoxide. The mixture of carbons obtained as a result of the reaction of hydrogenating the carbon dioxide may be used as a high-caloric fuel for power-generating units at the thermal power stations. The non-reacted carbon dioxide is fed back into the reactor system for hydrogenation.

**Example 2:** A mixture of liquid and gaseous hydrocarbons obtained in the process of hydrogenating the carbon dioxide (Example 1) is directed for separation, where the liquid hydrocarbons are separated from the gaseous ones. The gaseous hydrocarbons, containing (in percentage of the mass): C₁ - 8 %, C₂ - 15 %, C₃ - 35 %, C₄ - 42 %, are compressed up to pressure of 20,26 bar (20 atm) and directed for catalytic aromatization at temperature 450 °C. The aromatization reaction is carried out in the presence of a catalyst containing oxides of zinc and zirconium with a carrier of aluminium, zirconium and their oxides and phosphates.

As a result of the performed catalytic aromatization, the liquid hydrocarbons obtained in the process of hydrogenating the carbon dioxide represent 37 % of the mass.

The gaseous products of the reaction, including also CO₂, are directed to the fuel system of the thermal power station, and the fraction of liquid hydrocarbons is used as a high-octane component of the motor fuels.

**Example 3:** A gas mixture of carbon dioxide with hydrogen in the volumetric ratio of H₂/CO₂ = 4 is compressed to pressure 35,46 bar (35 atm) and heated up to temperature 300 °C and enters with volumetric speed 1,500 h⁻¹ a reactor with a catalyst, containing Fe, Mo and K, and carrier being a combination of aluminium, zirconium and their oxides and phosphates. The catalyst is subject to activation as it is described in Example 1. The catalyst and gas phase, which are in the reactor, are subject to the action of modulated high-frequency fields at frequency 15 MHz and modulation frequency 20 KHz. Subsequently, the process of hydrogenating the carbon dioxide is realized in the presence of plasma from single-electrode high-frequency discharges. In this case, the percentage of the hydrocarbons obtained is increased to 68 % of the mass, and at that the percentage of the liquid ones is 25 % of the mass. The obtained hydrocarbons and carbon monoxide (28 % of the mass) are directed to the fuel system of the thermal power station.

## Claims

1. A method of obtaining hydrocarbons from carbon dioxide, including a stage of obtaining carbon dioxide by adsorption with ethanolamine from flue gases with subsequent desorption and mixing with H₂ in volumetric ratio H₂/CO₂ equal to 4, with following compression up to pressure 35,46 - 50,66 bar (35 - 50 atm), heating up to 300 - 350 °C, and passing through a catalyst with volumetric speed 1,500 h⁻¹, **characterized by the fact that** the catalyst contains, in percentage of the mass, Fe 40 - 45 %, Mo 1.0 - 2.5 %, K 1.0 - 1.5 %, and a combination of aluminium, zirconium and their oxides and phosphates is used as a carrier.

2. A method according to claim 1, **characterized by the fact that** the gaseous hydrocarbons obtained in the process of hydrogenating the carbon dioxide are subject to catalytic aromatization.

3. A method according to claim 1, **characterized by the fact that** the gas mixture of H₂ and CO₂ and the catalyst in the process of hydrogenating the carbon dioxide at the first stage are subject to the action of modulated high-frequency fields in the frequency range from 1 MHz to 50 MHz at modulation frequency in the range from 1 KHz to 200 KHz, and at the second stage they are subject to action by plasma from single-electrode high-frequency discharges in the upper and central parts of the reactor.

## Patentansprüche

1. Verfahren zum Erhalt von Kohlenwasserstoffen aus Kohlendioxid, das eine Etappe von Kohlendioxidgewinnung aus Rauchgas durch die Adsorption mit Ethanolamin umfasst, wobei der benannten Adsorption eine Desorption und eine Mischung mit H₂ in einem volumetrischen H₂/CO₂-Verhältnis, das gleich 4 ist, folgen, wobei der benannten Mischung eine Komprimierung bis einem Druck von 35,46 bis 50,66 bar (von 35 bis 50 atm), eine Erwärmung bis Temperatur zwischen 300 und 350 °C, und ein Durchgang durch einen Katalysator mit der volumetrischen Geschwindigkeit von 1500 h⁻¹ folgen, **dadurch gekennzeichnet, dass** der Katalysator von 40 bis 45 % Fe, von 1,0 bis 2,5 % Mo, von 1,0 bis 1,5 % K, in Massenprozenten, enthält, **und dass** eine Kombination von Aluminium, Zirconium und ihren Oxiden und Phosphaten als einen Träger genutzt ist.

2. Verfahren nach Anspruch 1, **dadurch gekennzeichnet, dass** die im Prozess der Kohlendioxid-Hydrogenierung hergestellten gasförmigen Kohlenwasserstoffe einer katalytischen Aromatisierung unterworfen werden.

3. Verfahren nach Anspruch 1, **dadurch gekennzeichnet, dass** an der ersten Etappe im Prozess der Kohlendioxid-Hydrogenierung die gasförmige Mischung von H₂ und CO₂ und der Katalysator der Wirkung von modulierten Hochfrequenzfelder im Frequenzbereich von 1 MHz bis 50 MHz und mit modulierender Frequenz im Bereich von 1 KHz bis 200 KHz unterworfen werden **und dass** an der zweiten Etappe sie der Wirkung von Plasma der Hochfrequenzentladungen von einer Elektrode im Oberteil und Mittelteil des Reaktors unterworfen werden.

## Revendications

1. Procédé d'obtention d'hydrocarbures à partir de dioxyde de carbone comprenant une étape d'obtention du dioxyde de carbone provenant des fumées par adsorption avec éthanolamine étant suivie par désorption et mélangeage avec H₂ en un rapport volumétrique H₂/CO₂ égal à 4, ledit mélangeage étant suivi par une compression jusqu'à une pression de 35,46 à 50,66 bar (de 35 à 50 atm), un chauffage jusqu'à une température entre 300 et 350 °C, et un passage à travers un catalyseur à la vitesse volumétrique de 1500 h⁻¹, **caractérisé en ce que** le catalyseur contient, en pourcentage de masse, de 40 à 45 % de Fe, de 1,0 à 2,5 % de Mo, de 1,0 à 1,5 % de K, **et qu**'une combinaison d'aluminium, de zirconium et de leurs oxydes et phosphates est utilisée en tant que support.

2. Procédé selon la revendication 1, **caractérisé en ce que** les hydrocarbures gazeux obtenus dans le procédé d'hydrogénation du dioxyde de carbone sont soumis à une aromatisation catalytique.

3. Procédé selon la revendication 1, **caractérisé en ce qu'**à la première étape le mélange gazeux d'H₂ et de CO₂ et le catalyseur dans le procédé d'hydrogénation du dioxyde de carbone sont soumis à l'action des champs de haute fréquence modulés dont la fréquence est dans le domaine de fréquences de 1 MHz à 50 MHz 1 et la fréquence de modulation est dans le domaine de fréquences de 1 KHz à 200 KHz **et qu**'à la deuxième étape ils sont soumis à l'action de plasma provenant de décharges haute fréquence d'une électrode dans les parties supérieure et centrale du réacteur.
